# EUROPEAN PATENT APPLICATION

(11) **EP 2 113 573 A2**
(43) Date of publication of application: **04.11.2009**
(21) Application number: 09157644.7
(22) Date of filing: 08.04.2009
(51) Int. Cl.: C12Q 1/68

(54) **Environmental monitoring**

(30) Priority: 12.04.2008 GB 0806676
(71) Applicant: The Environment Agency, Aztec West Bristol Avon BS32 4UD (GB)
(72) Inventor: Bonsor, Robert William, Almondsbury Bristol, BS32 4UD (GB); Porter, Jonathan David, Almondsbury Bristol, BS32 4UD (GB)
(74) Representative: Turner, Rhiannon Rosalind

(57) **Abstract**

A process for monitoring contamination of water, said process comprising a method for detecting cellular material from a particular animal type in a sample, said method comprising detecting the presence therein of a nucleic acid having a sequence characteristic of DNA, and in particular mitochondrial DNA of said animal type, wherein the animal type is of a higher order than a single species.

## Description

### Field of Invention

The present invention relates to processes for monitoring contamination of water, for example processes associated with methods for tracking the source of waste material, in particular to the detection of faecal material in water, for example in river or sea water, which are liable to contamination, as well as to kits and reagents useful in the process.

### Background

Close monitoring of fresh, marine, surface and underground water presents a continuing challenge. Pollution can impact directly on ecosystems as well as the health of humans and other animals including fish, and on the environment as a whole. Today, a very wide range of contaminants is delivered to waters from a wide range of sources, including sewage effluent, sewage sludge, agricultural discharges or run-off, as well as industrial discharges. The sea, for example, may be contaminated by sewage effluent or industrial discharges directly, or rivers may deliver contaminants. The pollution may be within acceptable limits, or they may increase due to rainfall events, or accidental or deliberate discharges.

Pollution levels need to be continually monitored to ensure that levels do not rise to unacceptable heights, in particular in areas where human activity is carried out, such as at bathing beaches or rivers.

Sewage effluent presents a particular problem for monitoring. The sewage arises from a number of sources including humans and also farm animals. However, birds may also contribute to the levels of faecal pollution of waters such as fresh, marine, surface and underground water. Determining the host animal group of any pollution is important as this will allow appropriate steps to be taken to bring this under control.

Generally speaking, the source(s) of faecal contamination may be determined by the detection of specific bacterial DNA in the water. The nature of bacteria found in faecal matter is generally indicative of the source of that matter. Thus for example, faecal matter from humans contains a different bacterial profile than faecal matter derived from ruminants. Thus by determining the type of bacteria present in a sample, for example by detecting characteristic bacterial DNA, the source of any particular pollution, whether it be human sewage or agricultural waste, can be determined. Such procedures are described, for example, by Bernhard, A.E. and K. Field, 2000. Appl. Environ. Microbiol. 66,1587-1594, Bernhard, A.E., and K. Field, 2000. Appl. Environ. Microbiol. 66, 4571-4574, Dombek, P.E., et al., 2000. Appl. Environ. Microbiol. 66, 2572-2577 and Gawler, A., et al., 2007. Wat. Res. 41, 3780-3784.

However, such methods are limited. Birds, for example, may not necessarily contain a consistent profile of bacteria in their faeces and so bird waste cannot generally be positively identified in this way. Generally, the quantities of bacteria from bird faeces may be determined by identifying the amounts of human and ruminant sources, and making assumptions that the remainder will be largely bird related. Whilst relatively little may be done to control pollution by birds, this methodology does allow the potential that other polluting sources may escape detection.

Mitochondrial multiplex real-time PCR as a source tracking method in faecal-contaminated effluents was reported recently by Caldwell J. M. et al., Environmental Science & Technology (2007) 41, 9, 3277-3283. This paper demonstrated that species specific mitochondrial DNA from humans, swine and cows, could be identified in samples known to contain faecal matter from these sources. They suggest that this method could give rise to species-specific identification when used in combination with existing methods, but note problems associated with carry-over when species detected are carnivorous and also when there is a chance that the samples have been contaminated by non-faecal sources, such as skin, sputum and kitchen garbage. However, they have not addressed the specific problem of detecting pollution levels from broad genera or classes of avian sources in general. Rather they suggest that species specific identification is important.

### Summary of Invention

According to the present invention there is provided a process for monitoring contamination of water, said process comprising a method for detecting cellular material from a particular animal type in a sample, said method comprising detecting the presence therein of a nucleic acid having a sequence characteristic of DNA of said animal type.

As used herein, the expression "animal type" refers to an order higher than a species within the Phylum Chordata. In particular, it may be a Class of the Phylum such as Aves, Reptilia, Amphibia or fish classes such as Agnatha, Chondirchthyes or Osteichthyes, but it may also refer to Orders of Classes if required, such as Artiodactyla, or sub-orders such as Ruminantia or Arctoidea, or even families such as family Canidae, Cervidae, Bovidae or Suidae. Therefore, the detection of a nucleic acid having a sequence characteristic of DNA of said animal type indicates that the nucleic acid sequence is detectable in more than one species of the animal type, preferably in at least 2, 3, 4, 5, 6, 7, 8, 9 or 10 species of the animal type, more preferably in all known species of the animal type.

Detecting cellular material from a particular animal type in this way will provide important information about pollution levels. In particular, a major source of cellular material is faecal material. The applicants have found that host cells, presumed to be mainly from the gut epithelium are shed with any faecal discharge. These small levels however give rise to detectable levels of DNA and in particular mitochondrial DNA, that is present in cells in high copy number in samples containing the faecal matter. It is known that multiple genomes per mitochondrion are often present (e.g. average=4.6 in humans); that there multiple mitochondria per cell (average =107 in humans), giving rise to approximately 500mtDNA per cell. Also, it has been estimated that 1x10¹⁰ epithelial cells are shed into the gut per day per person (Satoh & Kuroiwa 1991 Exp Cell Res 196:137-140; Buller et al., 2006 Lab Investigation 86:1052-1063). As a result, methods for determining this cellular material will provide a good indicator of the level of faecal contamination from that class in any water sample.

It is possible that other sources of cellular material may be present in any particular water sample, for example as a result of the presence of skin cells or the like, or in the case of a specific incident of for example, a dead animal in the water. However, such incidents would generally be transient in nature and, therefore, when the method is used in the context of a continuous monitoring procedure, the results will provide a picture of the levels of faecal contamination. Thus in a particular embodiment, the method will be repeated using samples taken from the same water source at different time points, for example over periods of days, weeks, months or even years, as part of a monitoring process.

Furthermore, whilst some carry-over of DNA may occur where carnivorous animals such as humans are contributing to the contamination, the impact of this on the results could be at least partially accounted for if the method were used as part of a overall monitoring scheme, which detected for example mitochondrial DNA of said sources, for example human, swine or bovine, as reported by Caldwell et al. supra., or by conventional bacterial detection and/or source-tracking methods. For instance, a sample showing a low level of Aves DNA but, when tested, a high level of human derived material, may simply mean that human sources are principally responsible for any contamination and that the avian material is present as a result of carry over of avian food species. However, where the converse is true, then it may be reasonable to conclude that avian sources are principally responsible for any contamination.

Thus the method provides a more accurate and positive approach for identifying the nature of the pollution, and complements existing methods. It does not rely entirely on identification of bacteria and so is less prone to error as a result of variation in bacterial populations. As a result, more effective control measures may be taken.

By selecting DNA such as mitochondrial DNA, which is characteristic of an animal type, rather than an animal species, the method provides a useful addition to the existing source-tracking methods. For example, it will allow contamination from, in particular, avian sources to be positively identified rather than just detected by deductive methods as determined previously. Furthermore, in cases such as avian contamination in particular, it may be more important to get a broad picture of the extent of the contamination from the bird population as a whole than to identify specific species which are giving rise to the contamination. For example, the investigator may be querying whether the pollution is bird, human or agricultural, rather than which specific species of bird (for example) is causing pollution. The class selected in any particular monitoring process will depend upon the nature of the local fauna and the purpose and requirements of the monitoring. For example, in some situations, detection of DNA characteristic of a family such as family Canidae, may in fact be attributable to a single species such as dog. However, an advantage of the present invention is that it will allow the assays developed for the dog to be transferred to other areas of the world, where contamination by wolves, for example, may also be detected without modification.

The samples are generally water samples, for example from fresh, marine, surface and underground water, but in particular may be marine and/or bathing waters.

These samples are suitably filtered to extract solid material from the water, and the material extracted treated to lyse any cells present and extract DNA. Such methods are conventional in the art and illustrated hereinafter.

As described above, the animal type is above a species level, and may be up to phylum level. Therefore, the DNA, in particular the mitochondrial DNA, is selected to be characteristic of a particular classification level. Thus for example, it may be used to determine the family of the animal, such as Canidae, or to determine class of phylum, such as Aves (birds). In such cases, the specific DNA sequence detected is a sequence which is conserved amongst DNA and in particular mitochondrial DNA of the type. The DNA sequence selected may be part of a gene sequence, or in may be a non-coding DNA sequence, provided only that it is conserved amongst the specific animal type being investigated.

Particular examples of genes which may be highly conserved and therefore useful in the method of the invention include conserved regions of cytochrome b or other genes which are used to determine phylogeny at the required level.

This will provide a general indication of the source of faecal contamination which may be suitable in specific circumstances, including many environmental monitoring methods. However, if a more refined analysis is required, then detection of species specific mitochondrial DNA may additionally be carried out.

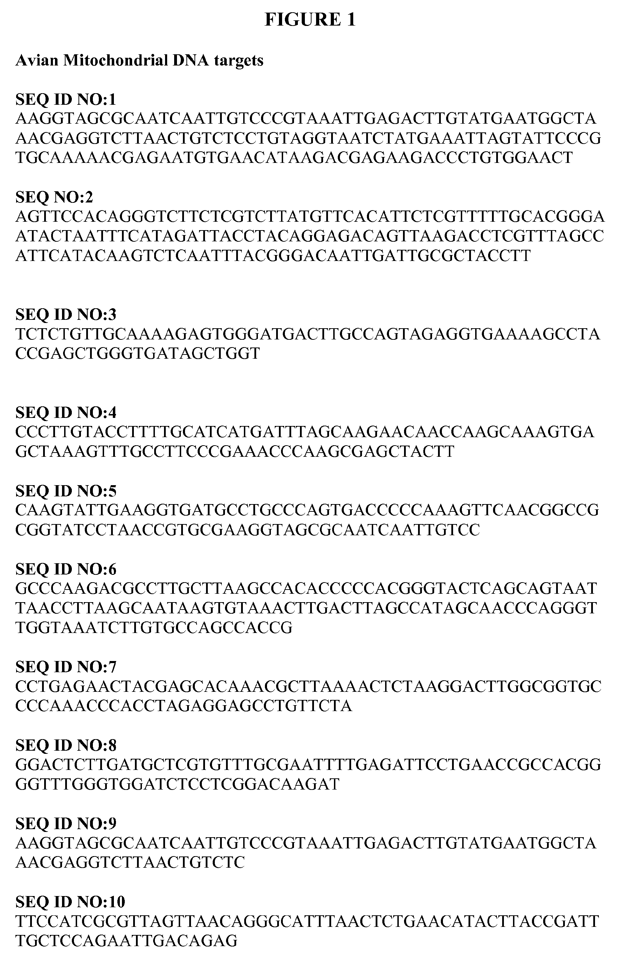

In a particular embodiment, the method is used to detect avian mitochondrial DNA. Suitable sequences include those shown in Figure 1 which are SEQ ID NOs:1 to 10. In a particular embodiment, the sequence detected as indicative of the presence and avian cellular matter is SEQ ID NO:1. However, characteristic fragments of these sequences, containing for example as few as 40 nucleotides may be detected provided the sequence is unique to avian mitochondrial DNA. The presence of significant amounts of these sequences will indicate the presence of avian faecal matter in the sample.

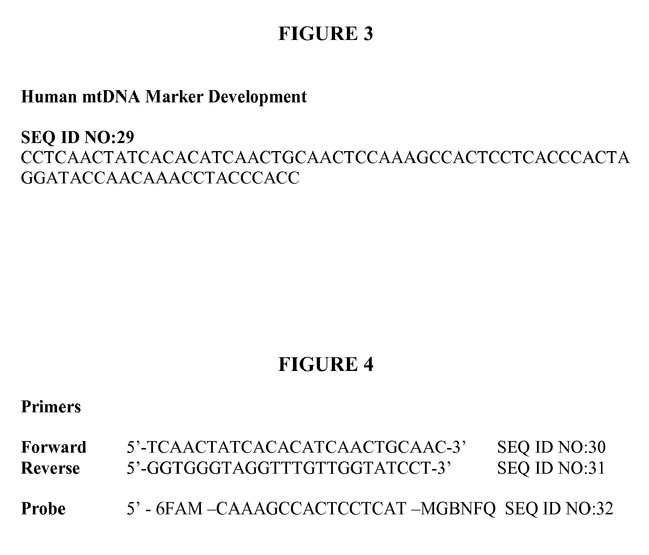

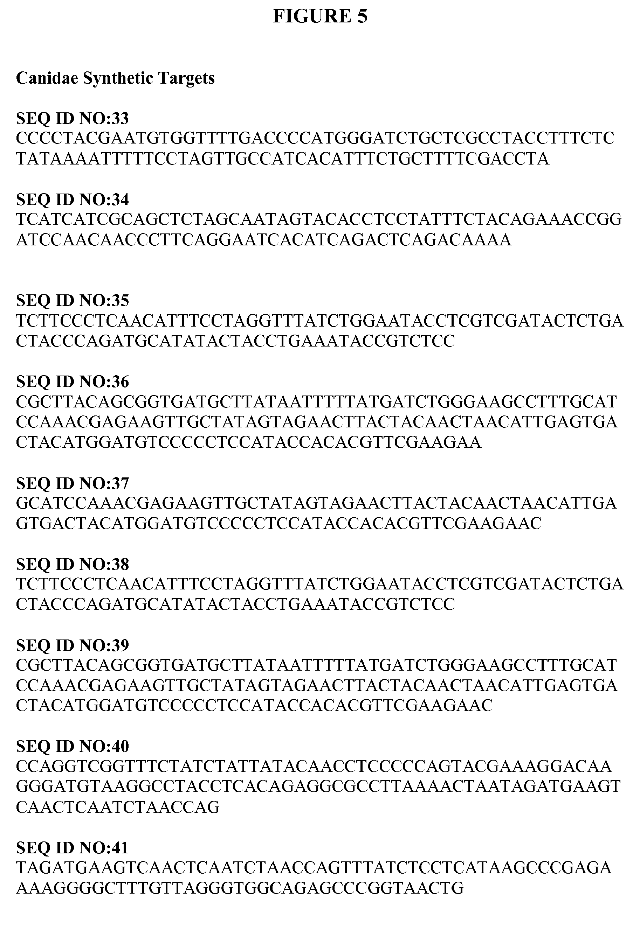

Additionally however, the identification of faecal contamination from other animal sources, including species such as human or dog, may be identified in a sample so as to allow contamination levels to be determined more generally. In the case of human, a particularly suitable sequence for detection is SEQ ID NO:29 in Figure 3. In the case of Canidae such as dogs, particularly suitable sequences for detection are SEQ ID NOs:33-41 as shown in Figure 5 and in particular, SEQ ID NO:36.

Detection of nucleic acids in the sample may be carried out using any of the available nucleic acid detection methods. In particular however, the detection will involve a DNA amplification step. Suitable amplification methods include polymerase chain reaction (PCR), ligase chain reaction (LCR), strand displacement amplification (SDA), transcription-mediated amplification (TMA), loop-mediated isothermal amplification (LAMP), rolling circle DNA amplification, multiplex ligation-dependent probe amplification (MLPA) and multiple displacement amplification. Such methods are well known in the art, and a skilled person would be in a position to adapt any of these to suit the method of the present invention.

Some of these methods may be carried out in a quantitative manner and this may be a particular embodiment of the present invention. Quantitation of the nucleic acid in the sample can provide information about the levels of the pollution from the particular animal type, which is useful in determining the overall pollution burden on the waters, and in the development of strategies for dealing with this.

Particular DNA amplification reactions which may be carried out quantitatively include PCR, MLPA and SDA.

In a particular embodiment, the DNA is detected using a PCR reaction, in particular a quantitative PCR reaction. Quantitative PCR is generally carried out by monitoring the progress of the reaction in real-time using a variety of signalling systems. These generally rely on the use of labels in particular fluorescent labels that may be monitored during the reaction. The labels are generally attached to oligonucleotides that act as probes or primers in an amplification reaction. Alternatively or additionally, the labels may be in the form of dyes which become associated with nucleic acids, for example by intercalating into double stranded nucleic acid, which fluoresce differently depending upon whether or not they are associated with a nucleic acid. Frequently, pairs of labels are used which interact with each other so as to modify the signal of one or both of these labels, depending upon the proximity to one another. The assay is arranged so that the proximity of the labels changes as an amplification reaction progresses. By monitoring the labels throughout, for example, by monitoring fluorescence and changes in fluorescence, the increases in the amplification product may be followed and this can be used to extrapolate to the copy number or amount of the sequence present in the initial sample.

Generic fluorescence PCR methods utilise DNA intercalating dyes that exhibit increased fluorescence when bound to double stranded DNA species. Fluorescence increase due to a rise in the bulk concentration of DNA during amplifications can be used to monitor reaction progress and to determine the initial target molecule copy number. These generic fluorescence PCR methods monitor the rise in bulk concentration of nucleic acids without any time penalty. A single fluorescent reading can be taken at the same point in every reaction. End point melting curve analysis can be used to discriminate artefacts from amplicon, as well as to discriminate amplicons. Peaks of products can be seen at concentrations that cannot be visualised by agarose gel electrophoresis.

Fluorescence PCR strand specific methods utilise additional nucleic acid reaction components to monitor the progress of amplification reactions. These methods may use fluorescence energy transfer (FET) as the basis of detection. One or more nucleic acid probes are labelled with fluorescent molecules, one of which is able to act as an energy donor and the other of which is an energy acceptor molecule. These are sometimes known as a reporter molecule and a quencher molecule respectively. The donor molecule is excited with a specific wavelength of light for which it will normally exhibit a fluorescence emission wavelength. The acceptor molecule is excited at this emission wavelength such that it can accept the emission energy of the donor molecule by a variety of distance-dependent energy transfer mechanisms. A specific example of fluorescence energy transfer that can occur is Fluorescence Resonance Energy Transfer or "FRET". Generally the acceptor molecule accepts the emission energy of the donor molecule when they are in close proximity (e.g. on the same, or a neighbouring molecule). The basis of FET or FRET detection is to monitor the changes at donor emission wavelength. Where the acceptor is also a fluorescent molecule, the acceptor emission wavelengths may also be monitored.

There are two commonly used types of FET or FRET probes, those using hydrolysis of nucleic acid probes to separate donor from acceptor and those using hybridisation to alter the spatial relationship of donor and acceptor molecules.

One particular assay for monitoring the progress of an amplification reaction is the TaqMan^{™} assay as described in US Patent No. 5,538,848. This assay utilizes hydrolysis probes consisting of DNA oligonucleotides, which are labelled with donor and acceptor molecules. The probes are designed to bind to a specific region on one strand of a PCR product. Following annealing of the PCR primer to this strand, Taq enzyme extends the DNA with 5' to 3' polymerase activity. Taq enzyme also exhibits 5' to 3' exonuclease activity. TaqMan^{™} probes are protected at the 3' end by phosphorylation to prevent them from priming Taq extension. If the TaqMan^{™} probe is hybridised to the product strand, then an extending Taq molecule may also hydrolyse the probe, liberating the donor from acceptor as the basis of detection. The signal in this instance is cumulative, the concentration of free donor and acceptor molecules increasing with each cycle of the amplification reaction.

Hybridisation probes are available in a number of guises. Molecular beacons are oligonucleotides that have complementary 5' and 3' sequences such that they form hairpin loops. Terminal fluorescent labels are in close proximity for FRET to occur when the hairpin structure is formed. Following hybridisation of molecular beacon to a complementary sequence the fluorescent labels are separated, so FRET does not occur, and this forms the basis of detection.

Pairs of labelled oligonucleotides may also be used as described for example in European Patent No. 0912760. These hybridise in close proximity on a PCR product strand, bringing donor and acceptor molecules together so that FRET can occur. Enhanced FRET is the basis of detection. Variants of this type include using a labelled amplification primer with a single adjacent probe.

An assay which utilises a combination of a single labelled probe and an intercalating dye is described in WO98/24548. WO99/42611 describes an assay that may be adapted to quantify the amount of the target sequence in the sample, using a combination of a set of nucleotides (at least one of which is fluorescently labelled) and a probe.

Using these techniques, it is possible to directly detect not only whether a particular target sequence is present within a sample (because amplification is taking place), but by appropriate manipulation of the data, to quantify the amount or determine when an amplification reaches completion. In particular, graphs plotting fluorescence against time or cycle number generally produces a sigmoidal plot peak, which rises steeply as amplification progresses and the amount of DNA in the sample increases, but which then reaches a plateau as amplification becomes saturated.

It has also been found that the properties of labels may be different depending upon their position within a nucleic acid sequence and, in particular, whether they are double or single stranded, located towards a terminus of an oligonucleotide, or positioned internally. The changes in the properties of the label when say it is attached to an end region of an oligonucleotide or primer, as compared to when it has been incorporated into a DNA sequence can itself give rise to a detectable signal. The principles and examples of how the technique operates are illustrated for example in WO/0079009 and WO02/057479.

The LUX (Light Upon extension) PCR chemistry is a particular example of this type of assay, and is known for example from Nazarenko et al., Nucleic Acids Res. 2002, 30(9) e 37, and Nucleic Acids Res. 2002, 30(9) p2089-2195, and exemplified for instance by Aitcihou et al. Molecular and Cellular Probes 19 (2005) 323-328. The technique is based upon the use of an oligonucleotide as a primer, which includes a modification so that it is detectable when hybridised to a complementary strand as compared to when it is free. In the usual mode, the method uses an oligonucleotide primer labelled with a single fluorescent moiety, to monitor nucleic acid amplification in the absence of traditional DNA-intercalating dye (such as SYBR dyes) or fluorescent probes. The primer used in LUX chemistry generally resembles that of a molecular beacon probe in that it relies on the formation of a hairpin motif to facilitate intra-molecular quenching in the absence of a target molecule. For example a LUX primer has a 5' motif (which is usually about 5 nucleotides in length) complementary to its 3' target specific priming terminus. When folded, the fluorescent moiety, which is generally bound to or close to a residue at the 3' end of the primer, is quenched via base-pairing or proximity to a residue, in particular a guanosine (dG) at the 5' end, and to some extent also by the nearest neighbour in the stem region. Generally, the label is attached to a cytosine (C) residue or a thymine (T) residue.

The reason for this is believed to be associated with the occurrence of a process of photoinduced electron transfer (PET). In the LUX process, a 3' amino-dC label is brought into close proximity with a 5' guanosine residue. The conformational microenvironment formed allows guanosine to behave as a ground state electron donor. This means that the near-by fluorophore fails to achieve the high-energy electron state required for photon emission. As such, quantum yield is decreased, thus quenching the dye.

When the guanosine is located internally of a nucleotide sequence, for example following extension of the primer and generation of the amplicon, the microenvironment of the fluorophore changes. The electron-donor effect is dissipated along the sequence, making the neighbouring guanosine residues poorer electron donors, so "de-quenching" the fluorophore by up to six fold. This change is sufficient to allow the real-time monitoring of the PCR reaction.

In the presence of target nucleic acid in the sample, the primer binds to a target recognition sequence (in the case of a hairpin, after linearization) and becomes extended so the label becomes internal within the sequence. As a result, it has been found that the quenching is reduced. The primer also acts as a conventional PCR primer and so the fluorescent moiety becomes incorporated into the amplicon. As the fluorescent moiety is not quenched, at least not to the same extent in the amplicon as compared to the hairpin primer, the existence of amplicon may be detected directly. There is no requirement for a separate probe and the primer may be a relatively simple and low cost molecule.

Any of these methods may be used to monitor the progress of a PCR and so allow for quantitation of the nucleic acid of the mitochondrial DNA in the sample. This amount can be used to quantify the level of pollution by the faecal material of the source animal in the source of the sample.

Further according to the present invention there is provided a kit for carrying out a method as described above, the kit comprising means for detecting mitochondrial DNA of an animal type.

Suitably, the kit will comprise reagents necessary to allow mitochondrial DNA of the animal type to be detected using a DNA amplification reaction, and in particular a PCR, especially a quantitative PCR reaction.

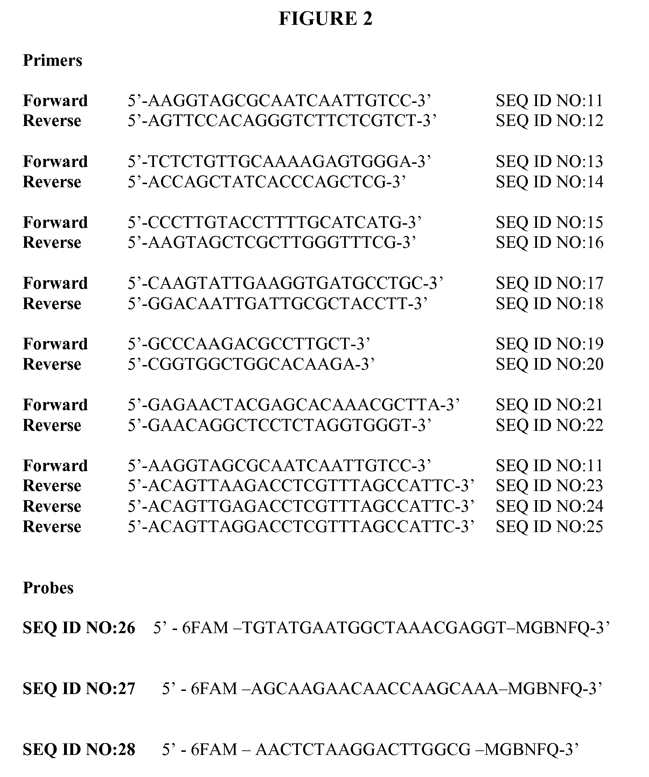

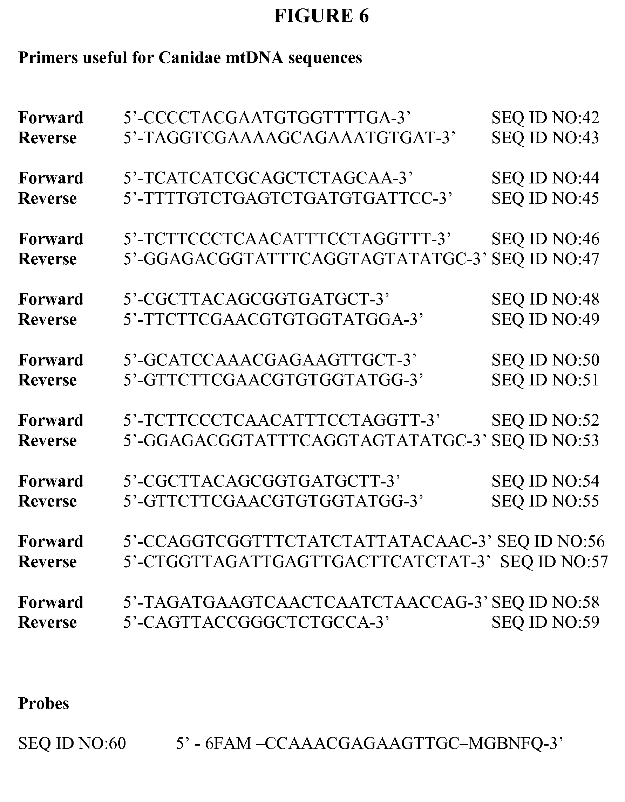

For example, the kit may comprise a pair of primers suitable for amplification of a DNA sequence such as a mitochondrial DNA sequence as described above. In particular, the primers are suitable for amplifying any one of SEQ ID NOs:1-10. Particularly suitable primers are shown in Figure 2 herein and listed as SEQ ID NOs:11-25. In this case, the kit is suitable for detection of avian mitochondrial DNA. In another embodiment, primers such as those of SEQ ID NOs:30 and 31 as illustrated in Figure 4 may also be provided in the kits, so that the avian contamination levels may be directly compared with the human levels, to allow an assessment to be made as to whether any contamination is directly from birds or is carry over in human faecal matter. Alternatively or additionally, the primers may comprise suitable combinations of the primers listed in Figure 6 as SEQ ID NOs:42-59 where the kit is intended to be able to identify Canidae contamination.

The kit suitably further comprises means for monitoring the progress of the amplification in real-time so as to allow for the quantitation of the nucleic acid in the sample. Such means are able to carry out any of the known monitored amplification reactions as outlined above and, in particular, may include fluorescent labels attached to an oligonucleotide or DNA intercalating dyes such as SYBRgreen or a combination of these.

In particular the means comprises a probe such as a TAQMAN^{™} probe which is able to hybridise to the target mitochondrial DNA sequence intermediate within the primers and which carries a pair of fluorescent labels which exchange fluorescent energy therebetween. In particular, one of the labels comprises a fluorescent energy donor and the other comprises a fluorescent energy acceptor molecule. In the case, the PCR is carried out using a polymerase with 5'-3'exonuclease activity so that bound probe is digested during the course of the amplification, thus releasing the fluorescent energy donor from the fluorescent energy acceptor molecule and so changing the resultant fluorescent signal. Suitable examples of such probes are listed in Figure 2 as SEQ ID NOs:26-28 for avian detection, the probe listed in Figure 4 as SEQ ID NO:32 for human detection and the probe listed in Figure 6 as SEQ ID NO:60 for Canidae contamination detection. In this case, a polymerase with 5'-3'exonuclease activity may form a further element of the kit.

The method described herein can be used in conjunction with other conventional methods to determine the source of all faecal material in a particular sample. For example, the relative amounts of faecal material from humans or ruminants may be determined by detecting characteristic bacteria or bacterial nucleic acid sequences in the sample, whilst the relative amounts of avian faecal material may be determined by the method described herein.

### Brief Description of Figures

The invention will now be particularly described by way of example with reference to the accompanying drawings in which:
Figure 1 illustrates a series of avian mitochondrial DNA sequences which may be detected in accordance with an embodiment of the invention;
Figure 2 illustrates a series of oligonucleotide primers and probes which may be used in detecting the sequences of Figure 1 by PCR;
Figure 3 illustrates a human mitochondrial DNA sequence which may be detected in accordance with an embodiment of the invention;
Figure 4 illustrates oligonucleotide primers and a probe which may be used in detecting the sequences of Figure 3 by PCR;
Figure 5 illustrates a series of Canidae mitochondrial DNA sequences which may be detected in accordance with an embodiment of the invention; and
Figure 6 illustrates a series of oligonucleotide primers and a probe which may be used in detecting the sequences of Figure 5 by PCR.

### Examples

### Example 1

A water sample (1litre), for example a sea water sample, is weighed, mixed by shaking and filtered through a cellulose acetate, cellulose nitrate, or mixed fibre membrane filter (0.45µm pore size) (this process may be repeated using a second membrane filter if the sample is particularly turbid.). All eluates are then combined and reweighed to determine the volume filtered.

Each filter is then placed into a sterile 55mm Petri dish. Guanidine thiocyanate (GITC) cell lysis buffer (1ml) is then pipetted onto the membrane filter and the Petri dish placed onto a see-saw rocking platform which is operated at 70rpm for 2-3 hours.

Ethanol (200µL, 95%) is pipetted onto the membrane filter. The surface of the membrane filter is then scraped for 1 minute using a sterile L-shaped spreader, turning the petri dish continuously at the same time. The membrane is then pushed to the side of the petri dish and squeeze out as much liquid as possible.

A sample (750µL) of liquid is removed from the petri dish and added to a labelled Qiagen DNEasy spin column. The column is centrifuged at 6000 g (approx. 9600 rpm in a Jouan microcentrifuge) for 1 minute and then placed in a collection tube to allow liquid to be removed. Any residual liquid in the petri dish is then added to the column which is spun again as before. Again liquid is removed into a clean collection tube. Qiagen wash buffer 1 (500µL) is then added to the column, which is spun at 6000g for 1 minute and liquid, removed to a collection tube. Qiagen wash buffer 2 (500µL) is added to the column, which is then spun at max. g for three minutes and liquid removed. Finally, Qiagen elution buffer (100µL) is added to the column and allowed to stand for at least 1 minute. After spinning at 6000 g for 1 minute, the eluate is collected into a microtube which is capped. The spin column and collection tubes are then discarded to be autoclaved.

The microtube containing a DNA preparation is, if necessary, stored in a labelled sealed bag at -20°C prior to analysis.

Analysis of DNA within the sample is then carried out by Quantative-Polymerase Chain Reaction (Q-PCR).

Q-PCR may be carried out using conventional procedures. In particular, the required primers at 800nM and where appropriate, Taqman probes (see Figures 2, 3 and 4) at 50nM are added to a TaqMan PCR master mix (available for example from Applied Biosystems). In all Figures, 6FAM indicates a Reporter dye and MGBNFQ indicates a Molecular-Groove Binding Non-Fluorescence Quencher. Where no TaqMan probe is available, other assay formats including in particular the use of SYBRgreen as a generic intercalating dye. The resultant mixture (23µl) is dispensed into a sterile well of, for example, a 96-well microtitre plate. Sample (1µL) prepared as described above is dispensed into the well using a clean tip. In addition, standard control oligonucleotides able to act as a control for the PCR are also added to a separate well, to provide a known number (10⁶) of a target for the amplification. PCR grade water is then added to the wells to make up the volume to 25µL per well. Other wells are used to produce control amplification standards using 10⁷ to 10⁴ control oligonucleotides.

The plate is then sealed with an optically clear 'Thermaseal' film using an electrically heated plate sealer. The plate is then spun for 2 minutes at 850 x g.

The plate is then loaded into a Q-PCR machine (such as a 7900HT TaqMan machine) and cycled in accordance with the manufacturers instructions. Results in the form of curves indicative of the rise in the amount of amplification target as well as the control target will be produced in the conventional manner. The curve for the control is checked to ensure that it is acceptable and that no inhibition has occurred.

Using this technique, the PCR reactions may been conducted using the following combination of primers/ probes illustrated in Figures 1 to 6 hereinafter to detect cellular material from Aves, Canidae and human sources, and the relationship of these to faecal contamination from these sources considered.

**Table 1**

| **Primers** | **Taqman Probe** | **Sequence Detected** | **Source** |
|---|---|---|---|
| Forward - SEQ ID NO:11 Reverse- SEQ ID NO:12 | SEQ ID NO:26 | SEQ ID NO:1 | Avian |
| Forward - SEQ ID NO:12 Reverse- SEQ ID NO:11 | SEQ ID NO:26 | SEQ ID NO:2 | Avian |
| Forward - SEQ ID NO:13 Reverse- SEQ ID NO:14 | SYBRgreen assay | SEQ ID NO:3 | Avian |
| Forward - SEQ ID NO:15 Reverse- SEQ ID NO:16 | SEQ ID NO:27 | SEQ ID NO:4 | Avian |
| Forward - SEQ ID NO:17 Reverse- SEQ ID NO:18 | SYBRgreen assay | SEQ ID NO:5 | Avian |
| Forward - SEQ ID NO:19 Reverse- SEQ ID NO:20 | SYBRgreen assay | SEQ ID NO:6 | Avian |
| Forward - SEQ ID NO:21 Reverse- SEQ ID NO:22 | SEQ ID NO:28 | SEQ ID NO:7 | Avian |
| Forward - SEQ ID NO:22 Reverse- SEQ ID NO:21 | SEQ ID NO:28 | SEQ ID NO:8 | Avian |
| Forward - SEQ ID NO:11 Reverse- SEQ ID NO:23 | SEQ ID NO:26 | SEQ ID NO:9 | Avian |
| Forward - SEQ ID NO:11 Reverse- SEQ ID NOs:23, 24, 25 degenerate primer | SEQ ID NO:26 | SEQ ID NO:10 | Avian |
| Forward - SEQ ID NO:30 Reverse- SEQ ID NO:31 | SEQ ID NO:32 | SEQ ID NO:29 | Human |
| Forward - SEQ ID NO:42 Reverse- SEQ ID NO:43 | SYBRgreen assay | SEQ ID NO:33 | Canidae/Dog |
| Forward - SEQ ID NO:44 Reverse- SEQ ID NO:45 | SYBRgreen assay | SEQ ID NO:34 | Canidae/Dog |
| Forward - SEQ ID NO:46 Reverse- SEQ ID NO:47 | SYBRgreen assay | SEQ ID NO:35 | Canidae/Dog |
| Forward - SEQ ID NO:48 Reverse- SEQ ID NO:49 | SEQ ID NO:60 | SEQ ID NO:36 | Canidae/Dog |
| Forward - SEQ ID NO:50 Reverse- SEQ ID NO:51 | SYBRgreen assay | SEQ ID NO:37 | Canidae/Dog |
| Forward - SEQ ID NO:52 Reverse- SEQ ID NO:54 | SYBRgreen assay | SEQ ID NO:38 | Canidae/Dog |
| Forward - SEQ ID NO:54 Reverse- SEQ ID NO:55 | SYBRgreen assay | SEQ ID NO:39 | Canidae/Dog |
| Forward - SEQ ID NO:56 Reverse- SEQ ID NO:57 | SYBRgreen assay | SEQ ID NO:40 | Canidae/Dog |
| Forward - SEQ ID NO:58 Reverse- SEQ ID NO:59 | SYBRgreen assay | SEQ ID NO:41 | Canidae/Dog |

The following results were obtained:

**Table 2. Example data of numbers of the mitochondrial DNA sequence numbers found in natural water samples.**

| Marker | Number of samples | Number of zero values | Average (St Dev) /100ml |
|---|---|---|---|
| Bird | 28 | 15 | 1.83x10⁴ (2.14x10⁴) |
| Canidae | 42 | 31 | 1.57x10³ (4.42x10³) |
| Human | 55 | 28 | 3.08x10³ (7.17x10³) |

Average Canidae mitochondrial sequence/100 ml data omits samples taken from a stream nearby a large kennels. Numbers in this stream were 4.7x10⁶/100 ml.

**Table 3. Example data of numbers of the mitochondrial sequences found in a sewage treatment works.**

| Stage | Human | Bird | Canidae |
|---|---|---|---|
| Influ | 4.95E+08 | 3.10E+07 | 7.35E+06 |
| Influ | 6.93E+08 | 2.18E+07 | 3.74E+06 |
| Influ | 3.33E+08 | 1.85E+06 | 4.37E+06 |
| Pre UV | 2.90E+07 | 5.33E+06 | 2.03E+05 |
| Pre UV | 1.94E+07 | 5.62E+06 | 2.74E+05 |
| Pre UV | 5.75E+07 | 9.44E+05 | 0.00E+00 |
| PostUV | 9.56E+06 | 4.82E+05 | 2.32E+05 |
| PostUV | 1.61E+07 | 2.83E+05 | 0.00E+00 |
| PostUV | 2.75E+07 | 4.14E+06 | 4.90E+05 |

| | | | |
|---|---|---|---|
| Influ = untreated influent; Pre UV = treated effluent, prior to UV disinfection; PostUV = treated effluent after UV disinfection. All numbers are /100ml | | | |

Faecal samples from known bird species were obtained from a range of sources across the UK. Similar samples were also obtained from other host animal groups (e.g. fish, seals). DNA preparations from all faecal sources were screened for the presence of all three mitochondrial markers. The results confirmed the expected specificity of the assays. The human mitochondrial marker was present only in human samples, and the Canidae mitochondrial marker was present only in dog samples. The bird mitochondrial marker was present in every known bird species and sample tested. It was absent from all dog (and human) mouth swabs, but was present in 50% of the human faecal samples, and in 29% of the dog faecal samples. The presence of the bird marker in these faecal samples was presumably as a result of diet. However, levels of the bird marker were less than those of the host animal marker levels.

The data shown in Tables 2 and 3 above demonstrate that of the samples tested, when present in a sample, average numbers of the bird marker sequence are greater than that of human or dog. A greater proportion of samples tested as positive for the bird and human marker sequences than for the dog sequence.

Data from bathing water samples (Table 2) show that when compared to a large sewage treatment works (serving approximately 160,000 population equivalents, with both domestic and industrial effluents from a large urban area - Table 3) the numbers of the markers are approximately 1000-fold less for the bird and human markers, and that average numbers of human marker are greater than average numbers of the bird marker in sewage. These data suggest the ability to distinguish pollution directly from birds (high levels of bird marker, low or undetectable levels of human marker) from that of sewage (high levels of human and bird marker).

## Claims

1. A process for monitoring contamination of water, said process comprising a method for detecting cellular material from a particular animal type in a sample, said method comprising detecting the presence therein of a nucleic acid having a sequence characteristic of DNA of said animal type.

2. A process according to claim 1 wherein the nucleic acid has a sequence characteristic of mitochondrial DNA of said animal type.

3. A process according to claim 1 or claim 2 wherein the animal type is Class Aves.

4. A process according to claim 3 wherein the DNA is one of SEQ NOs:1 to 10, or a characteristic fragment thereof.

5. A process according to claim 1 or 2 wherein the animal type is Family Canidae, and the DNA is one of SEQ ID NOs:33 to 41, or a characteristic fragment thereof.

6. A process according to any preceding claim wherein the nucleic acid is detected using a DNA amplification reaction which is a quantitative amplification reaction to allow the amount of DNA in the sample to be detected.

7. A process according to any one of the preceding claims wherein the presence of cellular material or faecal material from other animal sources is also determined.

8. A process according to claim 7 wherein the other animal source is a human source.

9. A process according to claim 8 wherein human cellular material is detected by detecting the presence of nucleic acid characteristic of human mitochondrial DNA.

10. A process according to any one of the preceding claims, wherein the method as defined in any one of claims 1 to 9 is repeated using samples from the same source over a period of time.

11. A kit for carrying out a process according to any one of the preceding claims, the kit comprising means for detecting a nucleic acid having a sequence characteristic of DNA of an animal type.

12. A kit according to claim 11 which comprise reagents necessary to allow mitochondrial DNA characteristic of the animal type to be detected using a DNA amplification reaction.

13. A kit according to claim 12 which comprises a pair of primers for amplifying said nucleic acid by means of a polymerase chain reaction.

14. A kit according to any one of claims 11 to 13 wherein the kit further comprises means to detect further nucleic acids, characteristic of other animal types or an animal species.

15. A kit according to any one of claims 11 to 14 further comprising means for monitoring the progress of the amplification in real-time so as to allow for the quantitation of the nucleic acid in the sample.


SEQUENCE LISTING

<110>  Environment Agency

<120>  Environmental Monitoring

<130>  RT/P1454EP00

<150>  GB 0806676.3
<151>  2008-04-12

<160>  60

<170>  PatentIn version 3.5

<210>  1
<211>  148
<212>  DNA
<213>  Aves

<400>  1
aaggtagcgc aatcaattgt cccgtaaatt gagacttgta tgaatggcta aacgaggtct      60

taactgtctc ctgtaggtaa tctatgaaat tagtattccc gtgcaaaaac gagaatgtga     120

acataagacg agaagaccct gtggaact                                        148


<210>  2
<211>  148
<212>  DNA
<213>  Aves

<400>  2
agttccacag ggtcttctcg tcttatgttc acattctcgt ttttgcacgg gaatactaat      60

ttcatagatt acctacagga gacagttaag acctcgttta gccattcata caagtctcaa     120

tttacgggac aattgattgc gctacctt                                        148


<210>  3
<211>  71
<212>  DNA
<213>  Aves

<400>  3
tctctgttgc aaaagagtgg gatgacttgc cagtagaggt gaaaagccta ccgagctggg      60

tgatagctgg t                                                           71


<210>  4
<211>  88
<212>  DNA
<213>  Aves

<400>  4
cccttgtacc ttttgcatca tgatttagca agaacaacca agcaaagtga gctaaagttt      60

gccttcccga aacccaagcg agctactt                                         88


<210>  5
<211>  90
<212>  DNA
<213>  Aves

<400>  5

caagtattga aggtgatgcc tgcccagtga cccccaaagt tcaacggccg cggtatccta 60

accgtgcgaa ggtagcgcaa tcaattgtcc 90

<210> 6
<211> 124
<212> DNA
<213> Aves

<400> 6
gcccaagacg ccttgcttaa gccacacccc cacgggtact cagcagtaat taaccttaag 60

caataagtgt aaacttgact tagccatagc aacccagggt tggtaaatct tgtgccagcc 120

accg 124

<210> 7
<211> 79
<212> DNA
<213> Aves

<400> 7
cctgagaact acgagcacaa acgcttaaaa ctctaaggac ttggcggtgc cccaaaccca 60

cctagaggag cctgttcta 79

<210> 8
<211> 79
<212> DNA
<213> Aves

<400> 8
ggactcttga tgctcgtgtt tgcgaatttt gagattcctg aaccgccacg gggtttgggt 60

ggatctcctc ggacaagat 79

<210> 9
<211> 70
<212> DNA
<213> Aves

<400> 9
aaggtagcgc aatcaattgt cccgtaaatt gagacttgta tgaatggcta aacgaggtct 60

taactgtctc 70

<210> 10
<211> 70
<212> DNA
<213> Aves

<400> 10
ttccatcgcg ttagttaaca gggcatttaa ctctgaacat acttaccgat ttgctccaga 60

attgacagag 70

<210> 11
<211> 22
<212> DNA
<213> Artificial

<220>
<223> Primer sequence

<400>  11
aaggtagcgc aatcaattgt cc                                                22

<210>  12
<211>  23
<212>  DNA
<213>  Artificial

<220>
<223>  Primer sequence

<400>  12
agttccacag ggtcttctcg tct                                               23

<210>  13
<211>  22
<212>  DNA
<213>  Artificial

<220>
<223>  Primer sequence

<400>  13
tctctgttgc aaaagagtgg ga                                                22

<210>  14
<211>  20
<212>  DNA
<213>  Artificial

<220>
<223>  Primer sequence

<400>  14
accagctatc acccagctcg                                                   20

<210>  15
<211>  22
<212>  DNA
<213>  Artificial

<220>
<223>  Primer sequence

<400>  15
cccttgtacc ttttgcatca tg                                                22

<210>  16
<211>  21
<212>  DNA
<213>  Artificial

<220>
<223>  Primer sequence

<400>  16
aagtagctcg cttgggtttc g                                                 21

<210>  17
<211>  23
<212>  DNA
<213>  Artificial

<220>
<223>  Primer sequence

<400>  17
caagtattga aggtgatgcc tgc                                              23


<210>  18
<211>  22
<212>  DNA
<213>  Artificial

<220>
<223>  Primer sequence

<400>  18
ggacaattga ttgcgctacc tt                                               22


<210>  19
<211>  17
<212>  DNA
<213>  Artificial

<220>
<223>  Primer sequence

<400>  19
gcccaagacg ccttgct                                                     17


<210>  20
<211>  17
<212>  DNA
<213>  Artificial

<220>
<223>  Primer sequence

<400>  20
cggtggctgg cacaaga                                                     17


<210>  21
<211>  24
<212>  DNA
<213>  Artificial

<220>
<223>  Primer sequence

<400>  21
gagaactacg agcacaaacg ctta                                             24


<210>  22
<211>  22
<212>  DNA
<213>  Artificial

<220>
<223>  Primer sequence

<400>  22
gaacaggctc ctctaggtgg gt                                               22


<210>  23

<211>  26
<212>  DNA
<213>  Artificial

<220>
<223>  Primer sequence

<400>  23
acagttaaga cctcgtttag ccattc                                            26


<210>  24
<211>  26
<212>  DNA
<213>  Artificial

<220>
<223>  Primer sequence

<400>  24
acagttgaga cctcgtttag ccattc                                            26


<210>  25
<211>  26
<212>  DNA
<213>  Artificial

<220>
<223>  Primer sequence

<400>  25
acagttagga cctcgtttag ccattc                                            26


<210>  26
<211>  22
<212>  DNA
<213>  Artificial

<220>
<223>  Probe sequence

<400>  26
tgtatgaatg gctaaacgag gt                                                22


<210>  27
<211>  20
<212>  DNA
<213>  Artificial

<220>
<223>  Probe sequence

<400>  27
agcaagaaca accaagcaaa                                                   20


<210>  28
<211>  18
<212>  DNA
<213>  Artificial

<220>
<223>  Probe sequence

<400>  28
aactctaagg acttggcg                                                     18

<210>  29
<211>  74
<212>  DNA
<213>  Homo sapiens

<400>  29
cctcaactat cacacatcaa ctgcaactcc aaagccactc ctcacccact aggataccaa      60

caaacctacc cacc                                                        74


<210>  30
<211>  25
<212>  DNA
<213>  Artificial

<220>
<223>  Primer sequence

<400>  30
tcaactatca cacatcaact gcaac                                            25


<210>  31
<211>  24
<212>  DNA
<213>  Artificial

<220>
<223>  Primer sequence

<400>  31
ggtgggtagg tttgttggta tcct                                             24


<210>  32
<211>  16
<212>  DNA
<213>  Artificial

<220>
<223>  Probe sequence

<400>  32
caaagccact cctcat                                                      16


<210>  33
<211>  97
<212>  DNA
<213>  Canidae

<400>  33
cccctacgaa tgtggttttg accccatggg atctgctcgc ctacctttct ctataaaatt      60

tttcctagtt gccatcacat ttctgctttt cgaccta                               97


<210>  34
<211>  92
<212>  DNA
<213>  Canidae

<400>  34
tcatcatcgc agctctagca atagtacacc tcctatttct acagaaaccg gatccaacaa      60

cccttcagga atcacatcag actcagacaa aa                                    92

<210>  35
<211>  89
<212>  DNA
<213>  Canidae

<400>  35
tcttccctca acatttccta ggtttatctg gaatacctcg tcgatactct gactacccag      60

atgcatatac tacctgaaat accgtctcc                                        89


<210>  36
<211>  140
<212>  DNA
<213>  Canidae

<400>  36
   cgcttacagc ggtgatgctt ataattttta tgatctggga agcctttgca tccaaacgag      60
   aagttgctat agtagaactt actacaacta acattgagtg actacatgga tgtccccctc      120
   cataccacac gttcgaagaa                                                 140


<210>  37
<211>  94
<212>  DNA
<213>  Canidae

<400>  37
gcatccaaac gagaagttgc tatagtagaa cttactacaa ctaacattga gtgactacat      60

ggatgtcccc ctccatacca cacgttcgaa gaac                                  94


<210>  38
<211>  89
<212>  DNA
<213>  Canidae

<400>  38
tcttccctca acatttccta ggtttatctg gaatacctcg tcgatactct gactacccag      60

atgcatatac tacctgaaat accgtctcc                                        89


<210>  39
<211>  141
<212>  DNA
<213>  Canidae

<400>  39
   cgcttacagc ggtgatgctt ataattttta tgatctggga agcctttgca tccaaacgag      60
   aagttgctat agtagaactt actacaacta acattgagtg actacatgga tgtccccctc      120
   cataccacac gttcgaagaa c                                               141


<210>  40
<211>  117
<212>  DNA
<213>  Canidae

<400>  40
ccaggtcggt ttctatctat tatacaacct cccccagtac gaaaggacaa gggatgtaag      60

gcctacctca cagaggcgcc ttaaaactaa tagatgaagt caactcaatc taaccag          117

<210>  41
<211>  87
<212>  DNA
<213>  Canidae

<400>  41
tagatgaagt caactcaatc taaccagttt atctcctcat aagcccgaga aaaggggctt          60

tgttagggtg gcagagcccg gtaactg                                              87

<210>  42
<211>  21
<212>  DNA
<213>  Artificial

<220>
<223>  Primer sequence

<400>  42
cccctacgaa tgtggttttg a                                                    21

<210>  43
<211>  24
<212>  DNA
<213>  Artificial

<220>
<223>  Primer sequence

<400>  43
taggtcgaaa agcagaaatg tgat                                                 24

<210>  44
<211>  21
<212>  DNA
<213>  Artificial

<220>
<223>  Primer sequence

<400>  44
tcatcatcgc agctctagca a                                                    21

<210>  45
<211>  25
<212>  DNA
<213>  Artificial

<220>
<223>  Primer sequence

<400>  45
ttttgtctga gtctgatgtg attcc                                                25

<210>  46
<211>  25
<212>  DNA
<213>  Artificial

<220>
<223>  Primer sequence

<400>  46
tcttccctca acatttccta ggttt                                          25


<210>  47
<211>  27
<212>  DNA
<213>  Artificial

<220>
<223>  Primer sequence

<400>  47
ggagacggta tttcaggtag tatatgc                                        27


<210>  48
<211>  19
<212>  DNA
<213>  Artificial

<220>
<223>  Primer sequence

<400>  48
cgcttacagc ggtgatgct                                                 19


<210>  49
<211>  22
<212>  DNA
<213>  Artificial

<220>
<223>  Primer sequence

<400>  49
ttcttcgaac gtgtggtatg ga                                             22


<210>  50
<211>  21
<212>  DNA
<213>  Artificial

<220>
<223>  Primer sequence

<400>  50
gcatccaaac gagaagttgc t                                              21


<210>  51
<211>  22
<212>  DNA
<213>  Artificial

<220>
<223>  Primer sequence

<400>  51
gttcttcgaa cgtgtggtat gg                                             22


<210>  52
<211>  24
<212>  DNA
<213>  Artificial

<220>
<223>  Primer sequence

<400>  52
tcttccctca acatttccta ggtt                                              24


<210>  53
<211>  27
<212>  DNA
<213>  Artificial

<220>
<223>  Primer sequence

<400>  53
ggagacggta tttcaggtag tatatgc                                           27


<210>  54
<211>  20
<212>  DNA
<213>  Artificial

<220>
<223>  Primer sequence

<400>  54
cgcttacagc ggtgatgctt                                                   20


<210>  55
<211>  22
<212>  DNA
<213>  Artificial

<220>
<223>  Primer sequence

<400>  55
gttcttcgaa cgtgtggtat gg                                                22


<210>  56
<211>  28
<212>  DNA
<213>  Artificial

<220>
<223>  Primer sequence

<400>  56
ccaggtcggt ttctatctat tatacaac                                          28


<210>  57
<211>  28
<212>  DNA
<213>  Artificial

<220>
<223>  Primer sequence

<400>  57
ctggttagat tgagttgact tcatctat                                          28


<210>  58

<211>  27
<212>  DNA
<213>  Artificial

<220>
<223>  Primer sequence

<400>  58
tagatgaagt caactcaatc taaccag                                          27


<210>  59
<211>  19
<212>  DNA
<213>  Artificial

<220>
<223>  Primer sequence

<400>  59
cagttaccgg gctctgcca                                                   19


<210>  60
<211>  16
<212>  DNA
<213>  Artificial

<220>
<223>  Probe sequence

<400>  60
ccaaacgaga agttgc                                                      16